# EUROPEAN PATENT APPLICATION

(11) **EP 1 688 434 A1**
(43) Date of publication of application: **09.08.2006**
(21) Application number: 04793073.0
(22) Date of filing: 21.10.2004
(51) Int. Cl.: C07K 16/18, A61K 39/395, G01N 33/53, G01N 33/543, G01N 33/577, C12N 5/12, C12N 15/06, C12P 21/08, C07K 16/42, A61P 5/00, A61P 25/00, A61P 1/00, A61P 3/04, A61P 3/10, A61P 9/06, A61P 9/10, A61P 9/12, A61P 15/00, A61P 25/08, A61P 25/16, A61P 25/20, A61P 25/22, A61P 25/24, A61P 25/28, A61P 25/32

(54) **ANTIBODY AND USE OF THE SAME**

(30) Priority: 22.10.2003 JP 2003361639
(71) Applicant: Takeda Pharmaceutical Company Limited, Osaka-shi, Osaka 5408645 (JP)
(72) Inventor: MATSUMOTO, Hirokazu c/o Tadeka Pharm Co Ld, Ibaraki 300-4293 (JP); NOGUCHI, Jiro c/o Takeda Pharm. Co Ltd, Tsukuba-shi, Ibaraki 300-4293 (JP); MASUDA, Yasushi c/o Takeda Pharm. Co Ltd, Tsukuba-shi, Ibaraki 300-4293 (JP)
(74) Representative: Lewin, John Harvey
(86) International application number: PCT/JP2004/015961
(87) International publication number: WO 2005/037870

(57) **Abstract**

The present invention provides a novel antibody useful in developing therapeutic, preventive and diagnostic agents for diseases associated with human ZAQL-2, a method of quantifying ZAQL-2 by using the antibody, etc. More particularly, the present invention provides an antibody reacting specifically with human ZAQL-2 or its derivatives, a method of quantifying ZAQL-2 by using the antibody, a pharmaceutical drug comprising the antibody, and so on.

## Description

### TECHNICAL FIELD

The present invention relates to a novel antibody reacting specifically with a polypeptide comprising the amino acid sequence represented by SEQ ID NO: 1, or salts thereof. More particularly, the present invention relates to a method of quantifying the polypeptide or salts thereof based on an antigen-antibody reaction, an antibody useful in developing an agent for diagnosis and prevention and/or treatment of diseases associated with the polypeptide or salts thereof, using its neutralizing activity, and so on.

### BACKGROUND ART

Human Bv8 maturation peptide (hereinafter sometimes briefly referred to as human ZAQL-2) has a structure similar to human ZAQ ligand-1 (human ZAQL-1) (WO 01/16309, WO 02/64835, WO 02/62996, etc.) that activates orphan receptors ZAQ and 15E, exhibits an ileum contracting activity as human ZAQ ligand-1 does, and binds to ZAQ and 15E to activate these receptors (WO 02/62944, etc.). It is further reported that human ZAQL-2 has a neuroprotective effect by activating MAP kinase and PI-3 kinase (Eur. J. Neuroscience, 13, 1694, 2001). Subsequently, these peptides are also reported to be prokineticin-1 (PK-1) and prokineticin-1 (PK-2) as new peptides found in the DNA database (Mol. Pharamacol., 59, 692, 2001). It is further reported that intracerebroventricular Bv8 peptide (ZAQL-2) in rats is localized within the rat suprachiasmatic nucleus, its expression induces a circadian change, which increases during the light period, and ZAQL-2 given intracerebroventricularly decreases the locomotor activity of the rats (Nature, 417, 405, 2002).

### DISCLOSURE OF THE INVENTION

In order to further elucidate the physiological functions of human ZAQL-2, it has earnestly been desired to develop the assay system for detecting and/or quantifying human ZAQL-2 in a simple manner with high sensitivity.

In order to solve the problems described above, the present inventors have made extensive investigations and as a result, have produced a plurality of monoclonal antibodies using human ZAQL-2 as an immunogen and found that human ZAQL-2 can be detected specifically with high sensitivity using these antibodies in combination. Thus, changes of human ZAQL-2 in biological components such as blood, cerebrospinal fluids, urine, etc. can be determined in a simple manner with high sensitivity.

That is, the present invention provides the following features and so on.
[1] A monoclonal antibody reacting specifically with a polypeptide comprising the amino acid sequence represented by SEQ ID NO: 1, or a salt thereof.
[2] The monoclonal antibody according to [1], which reacts specifically with a peptide comprising at least one sequence selected from the amino acid sequences of the 8th to 9th, 11th, 15th, 17th, 21st, 23rd, 25th to 28th, 30th, 34th, 36th to 37th, 39th to 40th, 44th to 46th, 48th, 52nd-53rd, 55th, 64th, 66th, 68th, 70th to 73rd, 75th to 76th and 78th to 81 st in the amino acid sequence represented by SEQ ID NO: 1.
[3] The monoclonal antibody according to [1], which does not recognize a polypeptide comprising the amino acid sequence represented by SEQ ID NO: 2 or SEQ ID NO: 3.
[4] The monoclonal antibody according to [1], which is labeled.
[5] The monoclonal antibody according to [1], which is shown by ZAL2-103a producible from the hybridoma shown by ZAL2-103 (FERM BP-8431).
[6] The monoclonal antibody according to [1], which is shown by ZAL2-106a producible from the hybridoma shown by ZAL2-106 (FERM BP-8432).
[7] The antibody according to [1], which has a neutralizing activity to a peptide having the amino acid sequence represented by SEQ ID NO: 1.
[7a] The antibody according to [7], which suppresses approximately 40 to 100% of the activities of the polypeptide in about 1-10 mol of an antibody concentration based on 1 mol of the polypeptide having the amino acid sequence represented by SEQ ID NO: 1, or a salt thereof.
[8] A pharmaceutical composition comprising the monoclonal antibody according to [1].
[9] A diagnostic agent comprising the monoclonal antibody according to [1].
[10] A method of quantifying a polypeptide comprising the amino acid sequence represented by SEQ ID NO: 1, which comprises using the monoclonal antibody according to [1].
[11] A method for diagnosis of disease associated with a polypeptide comprising the amino acid sequence represented by SEQ ID NO: 1, or a salt thereof, which comprises using the monoclonal antibody according to [1].
[12] A method of quantifying a polypeptide comprising the amino acid sequence represented by SEQ ID NO: 1, or a salt thereof in a test fluid, which comprises competitively reacting the monoclonal antibody according to [1] with a test fluid and a labeled form of the polypeptide comprising the amino acid sequence represented by SEQ ID NO: 1, or a salt thereof, and measuring a ratio of the labeled polypeptide or a salt thereof bound to the antibody.
[13] A method of quantifying a polypeptide comprising the amino acid sequence represented by SEQ ID NO: 1, or a salt thereof in a test fluid, which comprises reacting the monoclonal antibody according to [5] immobilized on a carrier, a labeled form of the monoclonal antibody according to [6] and a test fluid, and then assaying the activity of a labeling agent.
[14] A method of quantifying a polypeptide comprising the amino acid sequence represented by SEQ ID NO: 1, or a salt thereof in a test fluid, which comprises reacting the monoclonal antibody according to [6] immobilized on a carrier, a labeled form of the monoclonal antibody according to [5] and a test fluid, and then assaying the activity of a labeling agent.
[14a] A method of quantifying a polypeptide comprising the amino acid sequence represented by SEQ ID NO: 1, or a salt thereof in a test fluid, which comprises reacting the monoclonal antibody according to [1] immobilized on a carrier, a labeled form of the monoclonal antibody according to [1] (which is different from said monoclonal antibody immobilized on a carrier) and a test fluid, and then assaying the activity of a labeling agent.
[15] A hybridoma producing the monoclonal antibody according to [1].
[16] The hybridoma according to [15], which is shown by ZAL2-103 (FERM BP-8431) or ZAL2-106 (FERM BP-8432).
[17] A method of producing the monoclonal antibody according to [1], which comprises culturing the hybridoma according to [15] in vivo or in vitro and collecting the monoclonal antibody according to [1] from the body fluid or culture.
[18] The pharmaceutical according to [8], which is an agent for the prevention and/or treatment of central nervous system disorders, motor dysfunction or endocrine diseases.
[19] The diagnostic agent according to [9], which is a diagnostic agent for central nervous system disorders, motor dysfunction or endocrine diseases.
[20] An antibody reacting specifically with the binding site of the antibody according to [1] against a polypeptide comprising the amino acid sequence represented by SEQ ID NO: 1, or a salt thereof.
[21] An antibody reacting specifically with the binding site of the antibody according to [5] against a polypeptide comprising the amino acid sequence represented by SEQ ID NO: 1.
[22] An antibody reacting specifically with the binding site of the antibody according to [6] against a polypeptide comprising the amino acid sequence represented by SEQ ID NO: 1.
[23] A method of preventing and/or treating central nervous system disorders, motor dysfunction or endocrine diseases, which comprises administering to a mammal an effective dose of the antibody according to [1].
[24] Use of the antibody according to [1] to manufacture an agent for the prevention and/or treatment of central nervous system disorders, motor dysfunction or endocrine diseases.

### BRIEF DESCRIPTION OF THE DRAWINGS

FIG. 1 shows the assay results of antibody titers in antisera from mice immunized with human ZAQL-2, wherein symbol -◇- (-open diamond-) denotes mouse No. 1, -□- (-open square-) denotes mouse No. 2, -Δ- (-open triangle-) denotes mouse No. 3, -○- (-open circle-) denotes mouse No. 4, -◆- (-closed diamond-) denotes mouse No. 5, -■- (-closed square-) denotes mouse No. 6, -▲-(-closed triangle-) denotes mouse No. 7 and -●- (-closed circle-) denotes mouse No. 8.
FIG. 2 shows the state where human ZAQL-2-immunized mouse-derived hybridoma has produced the antibody (the results of absorption spectrometry).
FIG. 3 shows the state where human ZAQL-2-immunized mouse-derived hybridoma has produced the antibody (the results of absorption spectrometry).
FIG. 4 shows the state where human ZAQL-2-immunized mouse-derived hybridoma has produced the antibody (the results of absorption spectrometry).

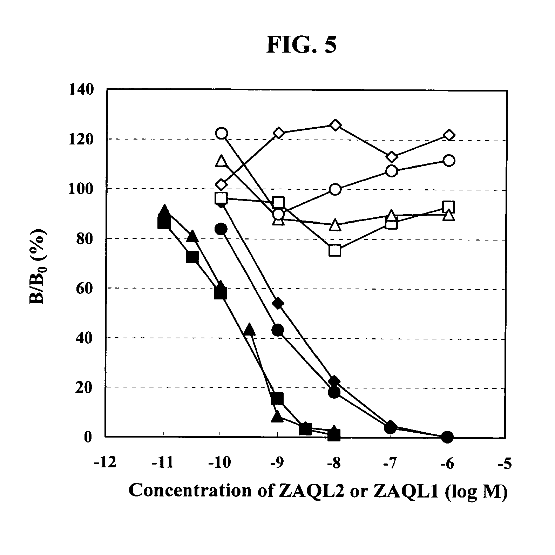

FIG 5 shows the reactivities of the antibody produced by human ZAQL-2-immunized mouse-derived hybridoma with human ZAQL-2 and human ZAQL-1 (the results of absorption spectrometry), wherein -◆- (-closed diamond-) denotes the reactivity of ZAL2-85a with human ZAQL-2, -◇- (-open diamond-) denotes the reactivity of ZAL2-85a with human ZAQL-1, -■- (-closed square-) denotes the reactivity of ZAL2-103a with human ZAQL-2, -□- (-open square-) denotes the reactivity of ZAL2-103a with human ZAQL-1, -▲- (-closed triangle-) denotes the reactivity of ZAL2-106a with human ZAQL-2, -Δ- (-open triangle-) denotes the reactivity of ZAL2-106a with human ZAQL-1, -●- (-closed circle-) denotes the reactivity of ZAL2-58a with human ZAQL-2 and -○- (-open circle-) denotes the reactivity of ZAL2-58a with human ZAQL-1.
FIG. 6 shows the results of ZAL2-103a and ZAL2-106a on human ZAQL-2 by competitive EIA, wherein -▲- (-closed triangle-) and -■- (-closed square-) denote the reactivities of ZAL2-103a and ZAL2-106a, respectively.
FIG 7 shows the results of the sandwich EIA using ZAL2-106a and ZAL2-103a-HRP, wherein -●- (-closed circle-) and -○- (-open circle-) denote the reactivities of human ZAQL-2 and human ZAQL-1, respectively. In the figure, non-specific absorbance (0.1>) of the assay system is shown as blank by □ (open square).

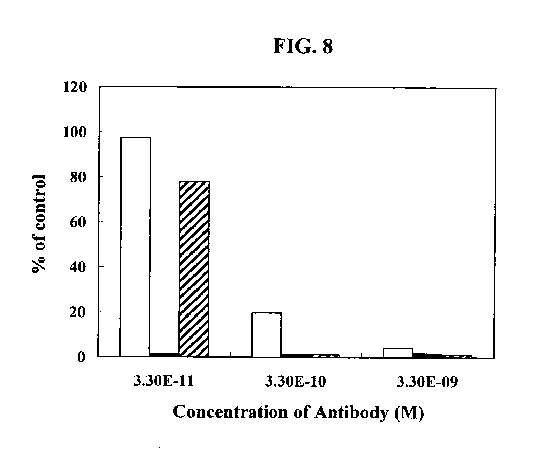

FIG. 8 shows the neutralizing action on the Ca²⁺ ion level increasing activity using I5E-expressed CHO cells in the presence of ZAL2-85a, ZAL2-103a or ZAL2-106a. The results show the intracellular Ca²⁺ ion level increasing activities after reacting human ZAQL-2 and each antibody at room temperature for an hour. In the figure, the ratios of the intracellular Ca²⁺ ion level increasing activities of ZAL2-85a, ZAL2-103a and ZAL2-106a in the presence of human ZAQL-2 as compared to the control (when no antibody was added) using the I5E-expressed CHO cells are shown as white, black and slant-shaded bars, respectively.
FIG 9 shows the eluted positions of the immunoreactivities of human ZAQL-2 in human plasma fractionated on a reversed phase HPLC. The arrow designates the eluted position of standard human ZAQL-2.

### BEST MODE FOR CARRYING OUT THE INVENTION

Throughout the specification, the proteins (polypeptides) are represented in accordance with the conventional way of describing peptides, that is, the N-terminus (amino terminus) at the left hand and the C-terminus (carboxyl terminus) at the right hand. In the protein used in the present invention including the polypeptide having the amino acid sequence represented by SEQ ID NO: 1, the C-terminus may be in any form of a carboxyl group, a carboxylate, an amide and an ester.

As the polypeptides comprising the amino acid sequence represented by SEQ ID NO: 1, there are used polypeptides having the amino acid sequence represented by SEQ ID NO: 1, wherein several (1 to 5) amino acids are added to the amino acid sequence described above; represented by SEQ ID NO: 1 or SEQ ID NO: 2 wherein several (1 to 5) amino acids are inserted into the amino acid sequence described above, those wherein several (1 to 5) amino acids in the amino acid sequence described above are replaced with other amino acids, and the like.

As salts of the polypeptide comprising the amino acid sequence represented by SEQ ID NO: 1, salts with physiologically acceptable acids (e.g., inorganic acids or organic acids), bases (e.g., alkali metal salts), etc. may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Examples of the monoclonal antibody, which specifically reacts with the polypeptide comprising the amino acid sequence represented by SEQ ID NO: 1 or salts thereof (hereinafter sometimes referred to as the antibody of the present invention), are monoclonal antibodies which specifically react with the polypeptide having the amino acid sequence represented by SEQ ID NO: 1, or a salt thereof, etc., preferably monoclonal antibodies which specifically react with the polypeptide having the amino acid sequence represented by SEQ ID NO: 1, or a salt thereof, etc.

Preferably, the antibody of the present invention specifically reacts with a peptide comprising at least one selected from the amino acids of the 8th to 9th, 11th, 15th, 17th, 21st, 23rd, 25th to 28th, 30th, 34th, 36th to 37th, 39th to 40th, 44th to 46th, 48th, 52nd-53rd, 55th, 64th, 66th, 68th, 70th to 73rd, 75th to 76th and 78th to 81st in the amino acid sequence represented by SEQ ID NO: 1, but does not recognize the polypeptide having the amino acid sequence represented by SEQ ID NO: 2 or SEQ ID NO: 3.

More preferably, the antibody of the present invention is an antibody that neutralizes the activities of the polypeptide having the amino acid sequence represented by SEQ ID NO: 1, or salts thereof. Specific examples include antibodies that the antibody concentration is approximately 1 to 10 mols, preferably about 1 mol, based on 1 mol of the polypeptide having the amino acid sequence represented by SEQ ID NO: 1, or salts thereof, and suppress the activities (e.g., the ZAQ binding activity, ZAQ activation, I5E binding activity, I5E activation, ileum contracting activity, MAP kinase activation, PI-3 kinase activation, etc.) of the polypeptide or salts thereof by approximately 40 to 100%, preferably approximately 60 to 100% and more preferably approximately 80 to 100%, and the like.

Specific examples include the monoclonal antibody shown by ZAL2-103a or ZAL2-106a, and the like.

Methods of preparing an antigen for the antibody of the present invention and methods of manufacturing the antibody are described below.

### (1) Preparation of antigen

To prepare the antibody of the present invention, any antigen such as (synthetic) peptides having 1 or 2 more antigenic determinants, which are the same as in the polypeptide comprising the amino acid sequence represented by SEQ ID NO: 1, or a salt thereof, etc. may be used (hereinafter these antigens are sometimes briefly referred to as the human ZAQL-2 antigen).

The polypeptide comprising the amino acid sequence represented by SEQ ID NO: 1, or a salt thereof can be produced by publicly known methods, e.g., the method as described in WO 02/62944. They may also be (a) prepared from mammalian tissue or cells of human, simian, rat, mouse, etc., by publicly known methods or with modifications, (b) chemically synthesized by publicly known peptide synthesis methods using a peptide synthesizer, etc., or (c) produced by culturing a transformant bearing a DNA encoding a polypeptide comprising amino acid represented by SEQ ID NO: 1, or a salt thereof.
(a) Where the human ZAQL-2 antigen is prepared from the mammalian tissues or cells, the tissues or cells are homogenized, then extracted with an acid, an alcohol, etc., and the extract is purified and isolated by a combination of salting-out, dialysis, gel filtration, chromatography techniques such as reverse phase chromatography, ion exchange chromatography, affinity chromatography and the like.
(b) Where the human ZAQL-2 antigen is prepared chemically, the synthetic peptides used are, for example, a peptide having the same structure as the human ZAQL-2 antigen purified from natural one, a peptide comprising 1 or 2 more amino acid sequences, which are the same amino acid sequences consisting of at least 3, preferably at least 6 amino acids in an optional region of the amino acid sequence represented by SEQ ID NO: 1, etc.
(c) Where the polypeptide comprising the amino acid sequence represented by SEQ ID NO: 1, or a salt thereof are produced using the DNA-bearing transformants, the DNA can be produced in accordance with publicly known cloning techniques [e.g., the method described in Molecular Cloning (2nd ed., J. Sambrook et al., Cold Spring Harbor Lab. Press, 1989), etc.]. The cloning techniques include (1) a method in which transformants bearing DNAs encoding the polypeptide comprising the amino acid sequence represented by SEQ ID NO:1, or a salt thereof are obtained from cDNA library by hybridization using DNA probes or DNA primers designed based on the amino acid sequence of the polypeptide comprising the amino acid sequence represented by SEQ ID NO:1, or a salt thereof, or (2) a method in which transformants bearing DNAs encoding the polypeptide comprising the amino acid sequence represented by SEQ ID NO: 1, or a salt thereof are obtained by PCR using DNA primers designed based on the amino acid sequence of the polypeptide comprising the amino acid sequence represented by SEQ ID NO: 1, or a salt thereof, etc.

Peptides used as the human ZAQL-2 antigen can be prepared (1) by peptide synthesis methods publicly known, or (2) by cleaving a polypeptide comprising the amino acid sequence represented by SEQ ID NO: 1 with an appropriate peptidase.

For the methods for peptide synthesis, for example, any of solid phase synthesis and liquid phase syntheses may be used. That is, the partial peptides or amino acids that can construct the peptide are condensed with the remaining part. Where the product contains protecting groups, these protecting groups are removed to give the desired peptide. Publicly known methods for condensation and removal of the protecting groups are methods described below.
(i) M. Bodanszky & M.A. Ondetti: Peptide Synthesis, Interscience Publishers, New York (1966)
(ii) Schroeder & Luebke: The Peptide, Academic Press, New York (1965)

After the reaction, the product may be purified and isolated by a combination of conventional purification methods such as solvent extraction, distillation, column chromatography, liquid chromatography, recrystallization, etc. to give the peptide. When the peptide obtained by the above methods is in a free form, the peptide can be converted into an appropriate salt by a publicly known method; conversely when the peptide is obtained in a salt form, it can be converted into a free form by a publicly known method.

Amides of the peptide may be obtained using commercially available resins for peptide synthesis, which are suitable for formation of the amides. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenylacetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective peptide according to various condensation methods publicly known in the art. At the end of the reaction, the peptide is cut out from the resin and at the same time, the protecting groups are removed to obtain the objective peptide. Alternatively, the objective peptide may also be obtained by protecting the peptide in part with chlorotrityl resin, oxime resin, 4-hydroxybenzoic acid type resin, etc., and removing the protective groups from the taken out peptide in a conventional manner.

For condensation of the protected amino acids described above, a variety of activation reagents for peptide synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the amino acids previously protected in the form of symmetric acid anhydrides, HOBt esters or HOOBt esters are activated, followed by adding the thus activated protected amino acids to the resin. Solvents suitable for use to activate the protected amino acids or condense with the resin may be appropriately chosen from solvents known to be usable for peptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; tertiary amines such as pyridine, etc.; ethers such as dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to peptide bond-forming reactions and is usually selected in the range of approximately -20°C to 50°C. The activated amino acid derivatives are used generally in an excess of about 1.5 to about 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel adverse effects on the subsequent reactions.

Examples of the protecting groups used to protect the amino groups in the starting amino acids include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc. Examples of the protecting groups for carboxyl groups include a C₁₋₆ alkyl group, C₃₋₈ cycloalkyl group, a C₇₋₁₄ aralkyl group, 2-adamantyl, 4-nitrobenzyl, 4-methoxybenzyl, 4-chlorobenzyl, phenacyl, benzyloxycarbonyl hydrazide, trityl hydrazide, or the like.

The hydroxyl group of serine and threonine can be protected through, for example, its esterification or etherification. Examples of the groups suitable for the esterification include a lower (C₁₋₆) alkanoyl group, such as acetyl group, etc.; an aroyl group such as benzoyl group, etc., and a group derived from carbonic acid such as benzyloxycarbonyl group, ethoxycarbonyl group, etc. Examples of a group suitable for the etherification include benzyl group, tetrahydropyranyl group, t-butyl group, etc.

Examples of groups for protecting the phenolic hydroxyl group of tyrosine include Bzl, Cl-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, Bom, Bum, Boc, Trt, Fmoc, etc.

Examples of the activated carboxyl groups in the starting compounds include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black, Pd-carbon, etc.; an acid treatment with anhydrous hydrofluoric acid, methanesulfonic acid, trifluoromethane-sulfonic acid or trifluoroacetic acid, or a mixture solution of these acids; a treatment with a base such as diisopropylethylamine, triethylamine, piperidine, piperazine, etc.; and reduction with sodium in liquid ammonia; or the like. The elimination of the protecting groups by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of the functional groups that should not be involved in the reaction of the starting materials, protecting groups, elimination of the protecting groups and activation of the functional groups involved in the reaction may be appropriately chosen from publicly known groups and publicly known means.

In another method for obtaining the amides of the peptide, for example, the α-carboxyl group of the carboxy terminal amino acid is first protected by amidation; the peptide chain is then extended to a desired length toward the amino group side. Thereafter, a peptide in which only the protecting group of the N-terminal α-amino group in the peptide chain has been eliminated from the peptide and a peptide (or amino acids) in which only the protecting group of the C-terminal carboxyl group has been eliminated are prepared. The two peptides are condensed in a mixture of the solvents described above. The details of the condensation reaction are the same as described above. After the protected peptide obtained by the condensation is purified, all the protecting groups are eliminated by the method described above to give the desired crude peptide. This crude peptide is purified by various known purification means. Lyophilization of the major fraction gives the amide of the desired peptide.

To prepare the esterified peptide, for example, the α-carboxyl group of the carboxy terminal amino acid is condensed with a desired alcohol to prepare the amino acid ester, which is followed by procedure similar to the preparation of the amidated peptide above to give the ester form of the desired peptide.

The human ZAQL-2 antigen may be provided for direct immunization in its immobilized form. The human ZAQL-2 antigen may also be bound or adsorbed to an appropriate carrier and the complex produced can be provided for immunization. A mixing ratio of the carrier to the human ZAQL-2 antigen (hapten) may be in any ratio of any type, as long as the antibody can be efficiently produced to the human ZAQL-2 antigen. A high molecular carrier conventionally used to produce an antibody to a hapten may be used in a weight ratio of 0.1 to 100 based on 1 of hapten. As such a high molecular carrier, there are used a naturally occurring high molecular carrier and a synthetic high molecular carrier. Examples of the naturally occurring high molecular carrier used are serum albumin from mammals such as bovine, rabbit, human, etc., thyroglobulins from mammals such as bovine, rabbit, etc., hemoglobins from mammals such as bovine, rabbit, human, sheep, etc or keyhole limpet KHL hemocyanin. Examples of the synthetic high molecular carrier, which can be used, are various latexes including polymers, copolymers, etc., for example, polyamino acids, polystyrenes, polyacryls, polyvinyls, polypropylenes, etc.

For coupling of the hapten and the carrier, a variety of condensing agents can be used. Examples of the condensing agents, which are advantageously employed, are diazonium compounds such as bis-diazotized benzidine capable of crosslinking tyrosines, histidines or tryptophans; dialdehyde compounds such as glutaraldehyde, etc. capable of crosslinking amino groups with each other; diisocyanate compounds such as toluene-2,4-diisocyanate, etc.; dimaleimide compounds such as N,N'-o-phenylenedimaleimide, etc. capable of crosslinking thiols with each other; maleimide activated ester compounds capable of crosslinking an amino group with a thiol group; carbodiimide compounds capable of crosslinking an amino group with a carboxyl group; etc. In the crosslinking of amino groups with each other, one amino group is reacted with an activated ester reagent (e.g., N-succinimidyl 3-(2-pyridyldithio)propionate (SPDP), etc.) having dithiopyridyl group and then reduced to introduce the thiol group, whereas another amino group is introduced with a maleimide group using a maleimide activated ester reagent, and the two groups may be reacted with each other.

### (2) Preparation of monoclonal antibody

The human ZAQL-2 antigen is administered to warm-blooded animal either solely or together with carriers or diluents to the site where the production of antibody is possible by administration routes such as intraperitoneally, intravenously, subcutaneously, etc. In order to potentiate the antibody productivity upon the administration, complete Freund's adjuvants or incomplete Freund's adjuvants may be administered. The administration is usually carried out once in every 2 to 6 weeks and approximately 2 to 10 times in total. Examples of the warm-blooded animal are monkeys, rabbits, dogs, guinea pigs, mice, rats, sheep, goats, fowl, etc. with mice being preferred for preparation of the monoclonal antibodies.

In preparing the monoclonal antibodies, the animal wherein the antibody titer is noted is chosen from warm-blooded animals, e.g., mice, immunized with the human ZAQL-2 antigen, then the spleen or lymph node is collected 2 to 5 days after the fmal immunization and antibody-producing cells contained therein are fused with myeloma cells, whereby the antibody-producing hybridomas of the present invention can be prepared. The anti-human ZAQL-2 antibody titer in antisera can be determined, for example, by reacting labeled human ZAQL-2, which will be described later, with the antiserum followed by assaying the binding activity of a labeling agent bound to the antibody. The fusion may be operated, for example, by known methods, e.g., by the Kohler and Milstein method [Nature, 256, 495 (1975)]. Examples of fusion accelerators are polyethylene glycol (PEG), Sendai virus, etc., of which PEG is preferably employed. Examples of the myeloma cells are NS-1, P3U1, SP2/0, AP-1, etc. In particular, P3U1 or the like is preferably employed. A preferred ratio in count of the antibody-producing cells (spleen cells) to the myeloma cells used is within a range of approximately 1:1 to 20:1. When PEG (preferably, PEG 1000 to PEG 6000) is added in a concentration of approximately 10 to 80% followed by incubation generally at 20 to 40°C, preferably at 30 to 37°C generally for 1 to 10 minutes, an efficient cell fusion can be carried out.

Various methods can be used for screening of the antibody-producing hybridomas of the present invention. Examples of such methods include a method which comprises adding the hybridoma supernatant to a solid phase (e.g., microplate) adsorbed with a polypeptide comprising the amino acid sequence represented by SEQ ID NO:1 or SEQ ID NO:2, its salts or partial peptides thereof, directly or together with a carrier, then adding an anti-immunoglobulin antibody (when mouse cells are used for the cell fusion, anti-mouse immunoglobulin antibody is used) labeled with a radioactive substance, an enzyme or the like, or Protein A and detecting the antibody of the present invention bound to the solid phase; a method which comprises adding the hybridoma supernatant to a solid phase adsorbed with an anti-immunoglobulin antibody or Protein A, adding a polypeptide comprising the amino acid sequence represented by SEQ ID NO:1 or SEQ ID NO:2, which is labeled with a radioactive substance, an enzyme, etc. and detecting the antibody of the present invention bound to the solid phase; etc. Screening and plating of the antibody of the present invention can be performed generally in a medium for animal cells (e.g., RPMI 1640) containing 10-20% fetal calf serum and supplemented with HAT (hypoxanthine, aminopterin and thymidine). The antibody titer in the hybridoma supernatant can be assayed by the same procedures as in the assay for the antibody titer of the antibody of the present invention in the antisera described above.

Separation and purification of the antibody of the present invention can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which involves collecting only the antibody with an activated adsorbent such as an antigen-binding solid phase, Protein A, Protein G, etc. and dissociating the binding to obtain the antibody; and the like].

As described above, the antibody of the present invention can be produced by culturing hybridoma cells in a warm-blooded animal in vivo or in vitro and collecting the antibody from the body fluids or the culture.

Screening can be performed for (a) the hybridomas that react with a partial region of polypeptide comprising the amino acid sequence represented by SEQ ID NO: 1 and (b) the hybridomas that react with the polypeptide described above but do not react with a segment of the polypeptide, for example, by measuring the binding property of a peptide corresponding to the segment to an antibody produced by the hybridoma.

Hereinafter, the method of quantifying (immunoassay for) the polypeptide comprising the amino acid sequence represented by SEQ ID NO: 1, or a salt thereof is described in more detail.

By using the antibody of the present invention, the polypeptide comprising the amino acid sequence represented by SEQ ID NO: 1 can be assayed, or can be detected by tissue staining, etc. For these purposes, the antibody molecule itself may be used, or F(ab')2, Fab' or Fab fractions of the antibody molecule may be used.

The quantification method using the antibody of the present invention is not particularly limited, but any quantification method can be used, so long as the amount of antibody, antigen or antibody-antigen complex corresponding to the amount of antigen (e.g., the amount of human ZAQL-2) in a fluid to be tested can be detected by chemical or physical means and the amount of the antigen can be calculated from a standard curve prepared from standard solutions containing known amounts of the antigen. Advantageously used are, for example, sandwich assay, competitive assay, immunometric assay and nephrometry; in terms of sensitivity and specificity, the sandwich assay described later is particularly preferred.

### (1) Sandwich assay

The polypeptide comprising the amino acid sequence represented by SEQ ID NO: 1 or a salt thereof is quantified by reacting the antibody of the present invention immobilized on a carrier with a labeled form of the antibody of the present invention and a test fluid, and assaying the activity of a labeling. Preferably, the method of quantifying the polypeptide comprising the amino acid sequence represented by SEQ ID NO: 1 or a salt thereof in a test fluid is characterized by reacting the antibody of the present invention immobilized on a carrier, a labeled form of the antibody of the present invention (which is an antibody different from the antibody of the present invention immobilized on a carrier) and a test fluid, and then assaying the activity of a labeling agent.

More preferred examples include:
(i) a method for quantification of a polypeptide comprising the amino acid sequence represented by SEQ ID NO: 1, or a salt thereof in a test fluid, which comprises reacting the monoclonal antibody shown by ZAL2-103a, which is immobilized on a carrier, a labeled form of the monoclonal antibody shown by ZAL2-106a and the test fluid, and then assaying the activity of a labeling agent;
(ii) a method for quantification of a polypeptide comprising the amino acid sequence represented by SEQ ID NO: 1, or a salt thereof in a test fluid, which comprises reacting the monoclonal antibody shown by ZAL2-106a, which is immobilized on a carrier, a labeled form of the monoclonal antibody shown by ZAL2-103a and the test fluid, and then assaying the activity of a labeling agent; and so on.

In the sandwich assay, a test fluid is reacted with an immobilized form of the antibody of the present invention (primary reaction), then reacted with a labeled form of the antibody of the present invention (secondary reaction) and the activity of a labeling agent on the insoluble carrier is assayed; thus, the amount of polypeptide comprising the amino acid sequence represented by SEQ ID NO: 1 (preferably human ZAQL-2), or a salt thereof in a test fluid can be assayed. The primary and secondary reactions may be carried out simultaneously or sequentially with intervals. The type of the labeling agent and the method of immobilization may be the same as those described hereinabove. In the immunoassay by the sandwich technique, it is not always necessary that the antibody used for the solid phase and for the labeled antibody should be one type or one species but a mixture of two or more antibodies may also be used for the purpose of improving the assay sensitivity, etc. In the sandwich assay method, where the antibody used in the primary reaction is, for example, the monoclonal antibody shown by ZAL2-103a, a preferred monoclonal antibody used in the secondary reaction is the monoclonal antibody shown by ZAL2-106a; where the antibody used in the primary reaction is the monoclonal antibody shown by ZAL2-106a, a monoclonal antibody preferably used in the secondary reaction is the monoclonal antibody shown by ZAL2-103a. Preferably, these antibodies are labeled with, e.g., horse radish peroxidase (HRP) and the labeled antibodies are provided for use.

### (2) Competitive assay

The antibody of the present invention, a test fluid and a labeled form of polypeptide comprising the amino acid sequence represented by SEQ ID NO: 1, or a salt thereof are competitively reacted, and a ratio of the labeled polypeptide comprising the amino acid sequence represented by SEQ ID NO: 1 bound to the antibody, or a salt thereof, is determined, thereby to quantify the polypeptide comprising the amino acid sequence represented by SEQ ID NO: 1, or a salt thereof in the test fluid.

The competitive assay is carried out by, e.g., a solid phase technique.

Specifically, anti-mouse IgG antibody (manufactured by ICN/CAPPEL) is used as a solid phase antibody, (i) the antibody of the present invention (e.g., ZAL2-103a or ZAL2-106a), (ii) a polypeptide comprising the amino acid sequence represented by SEQ ID NO: 1, or a salt thereof, which is labeled with HRP, and (iii) a test fluid are added to a plate where the solid phase antibody is present; after the reaction, the HRP activity adsorbed onto the solid phase is assayed to quantify the polypeptide comprising the amino acid sequence represented by SEQ ID NO: 1, or a salt thereof.

### (3) Immunometric assay

In the immunometric assay, an antigen in a test fluid and a solid phase antigen are competitively reacted with a given amount of a labeled form of the antibody of the present invention followed by separating the solid phase from the liquid phase; or an antigen in a test fluid and an excess amount of labeled form of the antibody of the present invention are reacted, then a solid phase antigen is added to bind an unreacted labeled form of the antibody of the present invention to the solid phase and the solid phase is then separated from the liquid phase. Thereafter, the labeled amount of any of the phases is measured to determine the antigen level in the test fluid.

### (4) Nephrometry

In the nephrometry, the amount of insoluble sediment, which is produced as a result of the antigen-antibody reaction in a gel or in a solution, is measured. Even when the amount of an antigen in a test fluid is small and only a small amount of the sediment is obtained, a laser nephrometry utilizing laser scattering can be suitably used.

Examples of labeling agents, which are employed for the aforesaid assay methods (1) to (4) using labeling agents, are radioisotopes, enzymes, fluorescent substances, luminescent substances, lanthanides, etc. Examples of radioisotopes are [¹²⁵I], [¹³¹I], [³H], [¹⁴C], etc. Preferred examples of the enzymes are those that are stable and have a higher specific activity, which include β-galactosidase, β-glucosidase, alkaline phosphatase, peroxidase, malate dehydrogenase, etc. Examples of the fluorescent substances include cyanine fluorescent dyes (e.g., Cy2, Cy3, Cy5, Cy5.5, Cy7 (manufactured by Amersham Biosciences), etc.), fluorescamine, fluorescein isothiocyanate, etc. Examples of the luminescent substances are luminol, a luminol derivative, luciferin, lucigenin, etc. Furthermore, a biotin-avidin system may be used as well for binding an antibody or antigen to a labeling agent.

In the immobilization of antigens or antibodies, physical adsorption may be used. Alternatively, chemical binding that is conventionally used for immobilization of proteins, enzymes, etc. may be used as well. Examples of the carrier include insoluble polysaccharides such as agarose, dextran, cellulose, etc.; synthetic resins such as polystyrene, polyacrylamide, silicone, etc.; or glass; and the like.

In applying each of these immunoassay techniques to the method of the present invention, it is not necessary to set any special condition, operation, etc. The assay system of the present invention may be constructed in addition to the conditions or operations conventionally used for each of the assay techniques, taking into account the technical consideration of one skilled in the art. For details of such conventional technical means, reference may be made to a variety of reviews, reference books, etc.[for example, Hiroshi Irie (ed.): "Radioimmunoassay" (published by Kodansha, 1974)]; Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immunochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)] (all published by Academic Press); etc.). Thus, where the assay system of the present invention is constructed by applying the sandwich immunoassay method, etc., its method is not limited to EXAMPLES later described.

As described above, the antibody of the present invention can quantify the polypeptide comprising the amino acid sequence represented by SEQ ID NO: 1, or a salt thereof with high sensitivity and hence, is useful for further elucidation of the physiological functions of the polypeptide described above and for diagnosis of diseases associated with the polypeptide described above. Specifically, the level of polypeptide comprising the amino acid sequence represented by SEQ ID NO: 1, or a salt thereof contained in body fluids (blood, plasma, serum, urine, etc.) is determined using the antibody of the present invention, whereby diagnosis can be given for, e.g., digestive diseases (e.g., enteritis, diarrhea, constipation, malabsorption syndrome, etc.), sleeping disorders (e.g., primary insomnia, circadian rhythm disorders (e.g., change in physical conditions caused by three-shift work, time zone change syndrome (jet lag), etc.), narcolepsy, etc.), seasonal depression, reproductive dysfunction (e.g., endometriosis, etc.), endocrine diseases, central nervous system disorders (e.g., senile dementia, Alzheimer's disease, parkinsonian syndrome, etc.), cerebral circulatory disorders (e.g., apoplexy, etc.), various disorders caused by aging, mental disorders (e.g., anxiety, depression, insomnia, schizophrenia, phobia, etc.), memory impairment, motor dysfunction (e.g., parkinsonian syndrome, etc.),epilepsy, alcoholism, hypertension, arteriosclerosis, arrhythmia, premenstrual syndrome, glaucoma, cancer(e.g., breast cancer, etc.), AIDS, diabetes mellitus, eating disorders (e.g., anorexia, bulimia, etc.), obesity(e.g., malignant mastocytosis, exogenous obesity, hyperinsulinar obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), osteoporosis, etc., preferably, sleeping disorders, reproductive dysfunction, endocrine diseases, central nervous system disorders, mental disorders, motor dysfunction, eating disorders, etc. For example, in diagnosis of sleeping disorders, the aforesaid ZAQL-2 in a body fluid is quantified and when the level of ZAQL-2 is more abundant than in healthy volunteers, e.g., its blood level is about 15 fmol/ml or more, preferably about 20 fmol/ml or more, it is diagnosed that one suffers from sleeping disorders.

In addition, the antibody of the present invention can be used as an agent for the prevention and/or treatment of diseases associated with the polypeptide comprising the amino acid sequence represented by SEQ ID NO: 1, or a salt thereof, for example, digestive diseases (e.g., enteritis, diarrhea, constipation, malabsorption syndrome, etc.), sleeping disorders (e.g., primary insomnia, circadian rhythm disorders (e.g., change in physical conditions caused by three-shift work, time zone change syndrome (jet lag), etc.), narcolepsy, etc.), seasonal depression, reproductive dysfunction (e.g., endometriosis, etc.), endocrine diseases, central nervous system disorders (e.g., senile dementia, Alzheimer's disease, parkinsonian syndrome, etc.), cerebral circulatory disorders (e.g., apoplexy, etc.), various disorders caused by aging, mental disorders (e.g., anxiety, depression, insomnia, schizophrenia, phobia, etc.), memory impairment, motor dysfunction (e.g., parkinsonian syndrome, etc.),epilepsy, alcoholism, hypertension, arteriosclerosis, arrhythmia, premenstrual syndrome, glaucoma, cancer(e.g., breast cancer, etc.), AIDS, diabetes mellitus, eating disorders (e.g., anorexia, bulimia, etc.), obesity (e.g., malignant mastocytosis, exogenous obesity, hyperinsulinar obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), osteoporosis, etc.; preferably as an agent for the prevention and/or treatment of sleeping disorders, reproductive dysfunction, endocrine diseases, central nervous system disorders, mental disorders, motor dysfunction, eating disorders, etc., and more preferably, as an agent for the prevention and/or treatment of endocrine diseases, central nervous system disorders, motor dysfunction, etc.

The preventive and/or therapeutic agent comprising the antibody of the present invention is low toxic, and can be administered orally or parenterally to human or mammals (e.g., rats, rabbits, sheep, swine, bovine, cats, dogs, monkeys, etc.) as it is in the form of liquid preparation or as a pharmaceutical composition of appropriate dosage form. The dose may vary depending on subject to be administered, target disease, conditions, route for administration, etc.; when it is used for the treatment of the adult patient with, e.g., eating disorders, it is advantageous to administer the antibody of the present invention parenterally to the patient through intravenous injection, normally in a single dose of approximately 0.01 to 20 mg/kg body weight, preferably about 0.1 to about 10 mg/kg body weight, and more preferably about 0.1 to about 5 mg/kg body weight, approximately 1 to 5 times, preferably approximately 1 to 3 times, per day. For oral administration, the corresponding dose may be administered. When the conditions are extremely serious, the dose may be increased depending on the conditions.

The antibody of the present invention may be administered directly in its intact form or in the form of an appropriate pharmaceutical composition. The pharmaceutical composition used for the administration described above may contain the antibody of the present invention or a salt thereof, a pharmacologically acceptable carrier and a diluent or an excipient. Such a pharmaceutical composition is provided in a dosage form suitable for oral or parenteral administration.

That is, examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by per se known methods. For example, the injectable preparations may be prepared by dissolving, suspending or emulsifying the antibody or a salt thereof described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate dissolution aid such as an alcohol [e.g., ethanol), a polyalcohol (e.g., propylene glycol, polyethylene glycol), a nonionic surfactant (e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a dissolution aid such as benzyl benzoate, benzyl alcohol, etc. The injection thus prepared is preferably filled in an appropriate ampoule. The suppository used for rectal administration may be prepared by blending the aforesaid antibody or a salt thereof with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the antibody contained is generally about 5 to about 500 mg per dosage unit form; it is preferred that the aforesaid antibody is contained in about 5 to about 100 mg especially in the form of injection, and in about 10 to 250 mg for the other forms.

Each composition described above may further contain other active components unless any adverse interaction is caused by formulating together with the antibody described above.

In the specification of the present invention, amino acids, etc. are shown by abbreviations and in this case, they are denoted by abbreviations in accordance with the IUPAC-IUB Commission on Biochemical Nomenclature or by the common codes in the art, examples of which are shown below. For amino acids that may have the optical isomer, L form is presented unless otherwise indicated.
- TFA: : trifluoroacetic acid
- DMF: : N,N-dimethylformamide
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- SPDP: : N-succinimidyl 3-(2-pyridyldithio)propionate
- GMBS: : N-(4-maleimidobutyryloxy)succinimide
- BSA: : bovine serum albumin
- BTG: :bovine thyroglobulin
- EIA: : enzyme immunoassay
- HPLC: : reversed phase high performance liquid chromatography
- HRP: horse radish peroxidase
- FBS: : fetal bovine serum
- d-FBS: :dialyzed fetal bovine serum
- TMB: 3,3',5,5'-tetramethylbenzidine
- H/HBSS: : HEPES buffered Hanks' balanced salt solution
- EDTA·Na: : disodium ethylenediaminetetraacetate dihydrate

The sequence identification numbers used in the present specification represent the amino acid sequences of the peptides below.

### [SEQ ID NO: 1]

This shows the amino acid sequence of human ZAQL-2.

### [SEQ ID NO: 2]

This shows the amino acid sequence of human ZAQL-1.

### [SEQ ID NO: 3]

This shows the amino acid sequence of human ZAQL-1. In the amino acid sequence represented by SEQ ID NO: 2, the 48th Val is replaced by Ile.

Hybridoma ZAL2-103 obtained in EXAMPLE 1 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-8431 since July 18, 2003.

Hybridoma ZAL2-106 obtained in EXAMPLE 1 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-8432 since July 18, 2003.

The antibodies acquired from the respective hybridomas are shown by the cell names with suffix "a."

Hereinafter, the present invention will be described in more detail, with reference to EXAMPLES but they are not deemed to limit the scope of the invention.

Human ZAQL-2 (SEQ ID NO: 1) used in EXAMPLES was obtained by the procedures described in REFERENCE EXAMPLE 1 of WO 02/62944.

Human ZAQL-1 (SEQ ID NO: 2) used in EXAMPLES was obtained by the procedures described in REFERENCE EXAMPLE 1 of WO 02/06483.

### EXAMPLE 1

### Preparation of anti-Human ZAQL-2 monoclonal antibody

### (1) Immunization

Human ZAQL-2 was subcutaneously given with a complete Freund's adjuvant to female BALB/C mice of 8 weeks old in a dose of approximately 80 µg/animal, respectively. These animals were boostered twice or thrice with an equal volume of the same immunogen every 3 weeks thereafter.

### (2) Preparation of horse radish peroxidase (HRP)-labeled human ZAQL-2

Human ZAQL-2 was conjugated to HRP (for enzyme immunoassay, manufactured by Boehringer Mannheim), which was used as the labeled antibody in the enzyme immunoassay (EIA).

After 8.5 mg (213 nmol) of HRP was dissolved in 0.02 M phosphate buffer (pH 6.8) supplemented with 0.95 ml of 0.1 M sodium chloride, the solution was mixed with 50 µl of DMF solution containing 1.99 mg of SPDP. The mixture was reacted at room temperature for 60 minutes. In addition, 0.5 ml of 0.1M acetate buffer (pH 4.5) containing 9.25 mg of dithiothreitol was added to the mixture, followed by reacting them at room temperature for 30 minutes. The reaction mixture was separated on a Sephadex G-25 column (eluant, 0.1 M phosphate buffer containing 2 mM EDTA, pH 6.0) to give SH-introduced HRP. Human ZAQL-2, 2 mg, was dissolved in 0.1 M phosphate buffer (pH 6.7) and the solution was mixed with 50 µl of DMF solution containing 0.76 mg (2.7 µmol) of GMBS. After reacting at room temperature for 60 minutes, the mixture was separated on a Sephadex G-25 column (eluant, 0.1 M phosphate buffer, pH 6.8) to give maleimido-introduced human ZAQL-2. After 1.67 mg (41.4 nmol) of the SH-introduced HRP and 1.2 mg (136 nmol) of the maleimide-introduced human ZAQL-1 thus produced were mixed with one another, the mixture was reacted at 4°C for a day. After completion of the reaction, the mixture was fractionated on Ultrogel AcA44 (manufactured by LKB-Pharmacia) column to give HRP-labeled human ZAQL-2.

### (3) Assay for antibody titer in antisera of mice immunized with human ZAQL-2

After boostering three times with human ZAQL-2 every three weeks, blood was withdrawn and collected from the fundus oculi one week after. The blood was centrifuged at 4°C and 12,000 rpm for 15 minutes and the supernatant was recovered to give antisera. The antibody titer in antisera was determined by the procedures described below. In order to prepare an anti-mouse immunoglobulin antibody-bound microplate, 100 µl each of 0.1 M carbonate buffer (pH 9.6) containing 100 µg/ml of anti-mouse immunoglobulin antibody (IgG fraction, Cappel Inc.) was dispensed onto a 96-well microplate, which was allowed to stand at 4°C for 24 hours. Next, the plate was washed with phosphate buffered saline (PBS, pH 7.4). To block redundant binding sites of the wells, 300 µl each of PBS containing 25% Block Ace (manufactured by Snow Brand Milk Products Co., Ltd.) was dispensed and then treated at 4°C for at least 24 hours.

After 50 µl of Buffer C [0.02 M phosphate buffer containing 1% BSA, 0.4 M NaCl and 0.05% 2 mM EDTA.Na (DOJINDO Co.), pH 7.0] and 100 µl of antisera to the complex diluted with Buffer C were added to each well of the anti-mouse immunoglobulin antibody-bound microplate obtained, the reaction was carried out at 4°C for 16 hours. Then, the plate was washed with PBS and 100 µl of the HRP-labeled human ZAQL-2 (diluted to 300-fold with Buffer C) prepared in (2) above was added and the reaction was carried out at room temperature for a day. Next, after the plate was washed with PBS, 100 µl of TMB (3,3',5,5'-tetramethylbenzidine) Microwell Peroxidase Substrate System (KIRKEGAARD & PERRY LAB, INC., consigned to Funakoshi Co., Ltd.) was added thereto and the reaction was carried out at room temperature for 10 minutes to assay the enzyme activity on a solid phase. The reaction was terminated by adding 100 µl of 1 M phosphoric acid and the absorption was then measured at 450 nm using a plate reader (BICHROMATIC, manufactured by Dainippon Pharmaceutical Co., Ltd.).

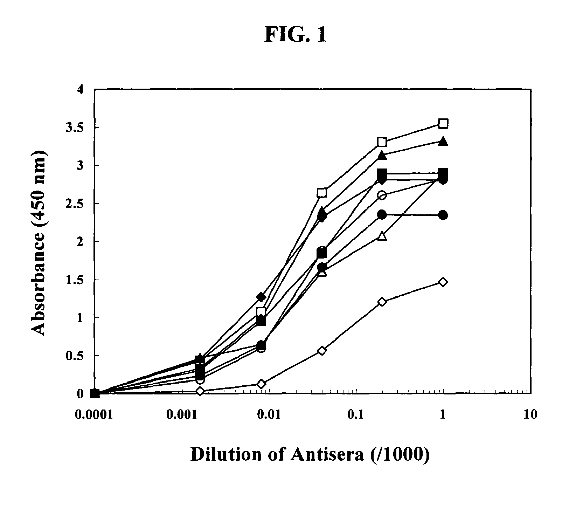

The results are shown in FIG 1.

An increase of the antibody titer to human ZAQL-2 was observed in the antisera to human ZAQL-2 in all of the 8 mice immunized.

### (4) Preparation of monoclonal anti-human ZAQL-2 antibody

A solution of 50-100 µg of the immunogen in 0.2 ml of physiological saline was intravenously inoculated to mice (No. 2 and No. 4) showing a relatively high antibody titer to achieve the final immunization. Four days after the final immunization, the spleen was withdrawn from the animal, pressed against a stainless mesh, filtered and suspended in Eagle's minimum essential medium (MEM) to give the spleen cell suspension. As cells used in cell fusion, BALB/C mouse-derived myeloma cell P3-X63.Ag8.U1 (P3U1) was used (Current Topics in Microbiology and Immunology, 81, 1, 1978).

The cell fusion was performed by a modification of the original method [Nature, 256, 495, 1975].

Spleen cells and P3U1 were washed 3 times with serum-free MEM, respectively, and they were blended to be in a 5:1 proportion of the spleen cells to P3U1 in cell counts. The mixture was centrifuged at 800 rpm for 15 minutes to deposit the cells. After the supernatant was thoroughly removed, the deposit was lightly loosened and 0.3 ml of 45% polyethylene glycol (PEG) 6000 (manufactured by Kochlight) was added thereto. The mixture was allowed to stand for 7 minutes in a warm water bath of 37°C to perform cell fusion. The fusion was followed by addition of MEM to the cells in a rate of 2 ml/min. When the added MEM reached 15 ml in total, the mixture was centrifuged at 600 rpm for 15 minutes and the supernatant was removed. The cell deposit was suspended in 10% fetal calf serum-containing GIT medium (Wako Pure Chemical Industries, Ltd.) (GIT-10% FCS) in 2 x 10⁵, and the suspension was plated on 192 wells of a 24-well Multidish (manufactured by Limbro) in 1 ml/well. After the plating, the cells were incubated at 37°C in a 5% carbonic acid incubator. Twenty-four hours after, GIT-10% FCS medium (HAT medium) containing HAT (1 x 10⁻⁴ M hypoxanthine, 4 x 10⁻⁷ M aminopterin and 1.6 x 10⁻³ M thymidine) was added to the cells in 1 ml/well, thereby to initiate HAT selective culture. The HAT selective culture was continued by discarding the old medium on Days 3, 6 and 9 after initiation of the incubation and instead, replenishing 1 ml of HAT medium. Growth of the hybridoma was noted on Days 9-14 after the cell fusion. When the culture medium turned yellow (ca. 1 x 10⁶ cells/ml), the supernatant was collected and the antibody titer was assayed by the procedures described in (3) above.

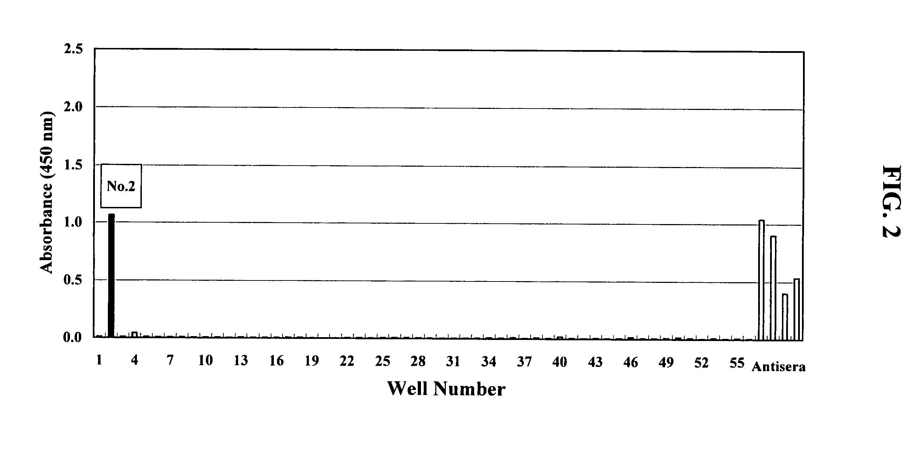

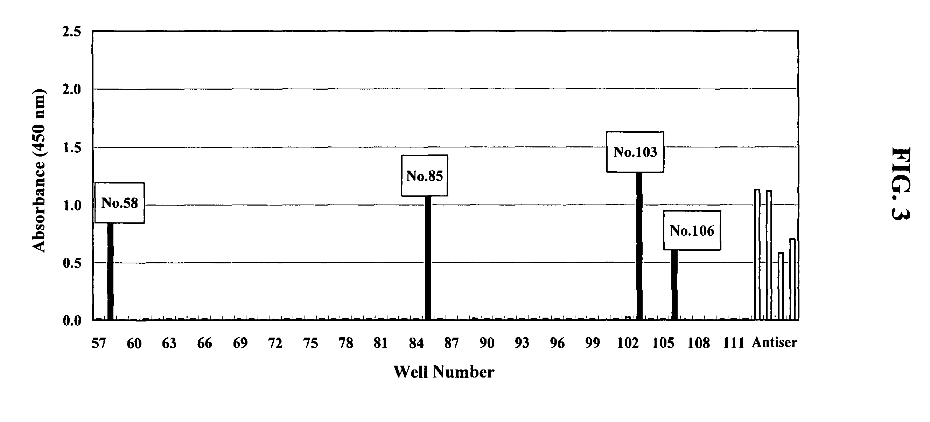

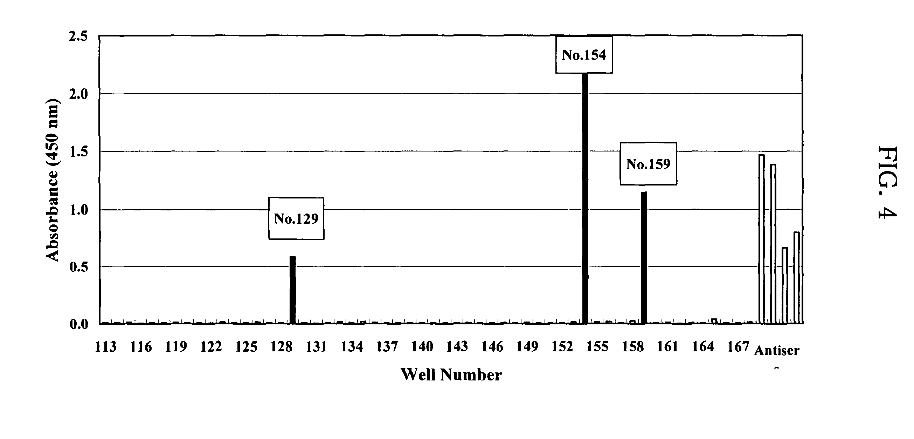

As examples where the antibody-producing cell lines of the hybridomas derived from the mice immunized with human ZAQL-2 were selected, the antibody-producing conditions of the hybridomas obtained by cell fusion using mice Nos. 2 and 4 (cf. FIG 1) are shown in FIGS. 2 to 4. The following four hybridomas in total were selected from the antibody-producing hybridomas acquired [TABLE 1].

**TABLE 1**

| Reactivity ¹⁾ | | | |
|---|---|---|---|
| Hybridoma No. | Human ZAQL-2 | Class/Subclass | Antibody |
| 1 | + | IgGl,K | ZAL2-103a |
| 2 | + | IgGl,K | ZAL2-106a |
| 3 | ± | IgG2b,K | ZAL2-85a |
| 4 | ± | IgGl,K | ZAL2-58a |

1) When 1 nM of human ZAQL-2 was present as an antigen:
   + :(B/B₀) < 0.50
   ± :0.50 ≦ (B/B₀) <0.70
   - :0.70 ≦ (B/B₀)
   B: The amount of HRP-labeled human ZAQL-2 bound to the antibody in the presence of the antigen
   B₀: The amount of HRP-labeled human ZAQL-2 bound to the antibody in the absence of the antigen

Next, these hybridomas were cloned by limiting dilution. In cloning, thymocytes from BALB/C mice were added as feeder cells in 5 x 10⁵ cells/well. After cloning, the hybridomas were intraperitoneally injected to mice (BALB/C) in 1 to 3 x 10⁶ cells/mouse, to which mice 0.5 ml of mineral oil had previously been given intraperitoneally. The ascites fluid containing the antibody was collected 6 to 20 days after.

The monoclonal antibody was purified from the ascites fluid obtained, using Protein A column. The ascites fluid, 6 to 20 ml, was diluted with an equal volume of binding buffer [1.5 M glycine containing 3.5 M NaCl and 0.05% NaN₃ (pH 9.0)], and the dilution was applied on recombinant Protein A-agarose (manufactured by Repligen) column, which had been previously equilibrated with the binding buffer. The specific antibody was eluted with an eluting buffer [0.1 M citrate buffer containing 0.05% NaN₃ (pH 3.0)]. The eluate was dialyzed to PBS at 4°C for 2 days, which was subjected to cell-free filtration through a filter of 0.22 µm (manufactured by Millipore) and then stored at 4°C or -80°C.

In determining the class/subclass of monoclonal antibodies, enzyme-linked immunosorbent assay (ELISA) using purified monoclonal antibody-bound solid phase was used. That is, 100 µl each of 0.1 M carbonate buffer (pH 9.6) solution containing 2 µg/ml of the antibody was dispensed on a 96-well microplate, which was allowed to stand at 4°C for 24 hours. Following the procedures described in (1) above, redundant binding sites of the wells were blocked with Block Ace. Thereafter, the class and subclass of the immobilized antibodies were determined by ELISA using an isotyping kit (Mouse-Typer™ Sub-Isotyping Kit, manufactured by Biorad). All of the four antibodies acquired had IgG1 for H chain and κ for L chain.

### EXAMPLE 2

### Competitive enzyme immunoassay

The reaction specificity was examined on the monoclonal antibodies prepared using human ZAQL-2-BTG as the immunogen by the following procedures.

First, the antibody titers of the four monoclonal antibodies in their solutions were assayed by the procedures described in EXAMPLE 1-(3) above, and the antibody concentration (ca. 30 to 50 ng/ml) wherein the binding amount in the labeled antibody reached about 50% of the saturation binding amount was determined as the antibody concentration used for competitive assay-EIA. Next, (i) 50 µl of the anti-human ZAQL-2 antibody solution diluted with Buffer C to have 50 ng/ml of each monoclonal antibody, (ii) 50 µl of the human ZAQL-1 solution or 50 µl of the human ZAQL-2 solution, diluted with Buffer C, and (iii) 50 µl of the HRP-labeled human ZAQL-2 (diluted to 400-fold with Buffer C) obtained in EXAMPLE 1-(3) described above, were added to the anti-mouse immunoglobulin antibody-bound microplate, followed by reacting them at 4°C for 16 hours. After the reaction, the plate was washed with PBS and the enzyme activity on the anti-mouse immunoglobulin antibody-bound microplate was assayed by the procedures described in EXAMPLE 1-(3).

The results are shown in FIG 5.

The results reveal that all of the antibodies reacted with human ZAQL-2 but they had no reactivity with human ZAQL-1.

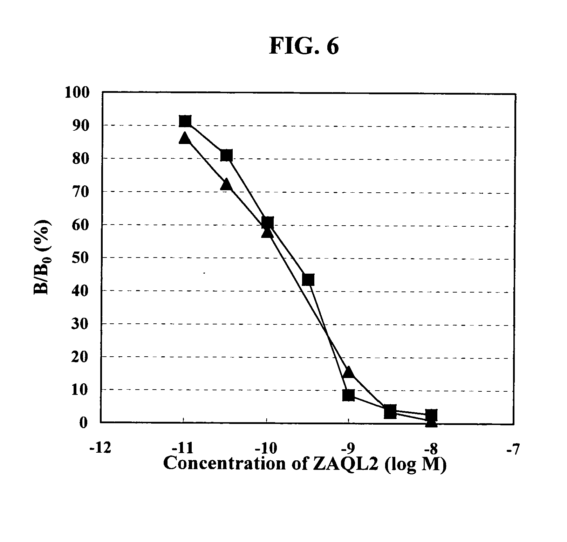

By way of illustration, the results of the competitive assay-EIA on the monoclonal antibodies ZAL2-103a and ZAL2-106a having the highest reactivity with human ZAQL-2 are shown in FIG. 6.

From the standard curves of ZAL2-103a and ZAL2-106a for human ZAQL-1, the concentration of human ZAQL-2, which gave the ratio to the highest reactivity (B/B₀)=0.5, was found to be 0.8 nM and 3 nM. Based on these results, ZAL2-103a and ZAL2-106a are indicative of a high reactivity with human ZAQL-2.

### EXAMPLE 3

### Preparation of HRP-labeled anti-human ZAQL-2 monoclonal antibody (ZAL2-103a-HRP)

After 50 µl of DMF containing 0.80 µmol of GMBS was added to 0.1 M phosphate buffer (pH 6.8) containing 9.98 mg (66.5 nmol) of the purified ZAL2-103a fraction, the mixture was reacted at room temperature for 40 minutes. The reaction solution was separated on a Sephadex G-25 column (eluant, 0.1 M phosphate buffer, pH 6.7) to give 6.99 mg of the maleimido-introduced antibody fraction. Next, 60 µl of DMF containing 6.42 µmol of SPDP was added to 1.14 ml of 0.02 M phosphate buffer containing 17.1 mg (428 nmol) of HRP (further containing 0.15 M NaCl) (pH 6.8), followed by reacting them at room temperature for 40 minutes. Subsequently, 0.4 ml of 0.1 M acetate buffer (pH 4.5) containing 64.2 µmol of dithiothreitol was added. After reacting at room temperature for 20 minutes, the reaction mixture was separated on a Sephadex G-25 column (eluant, 0.1 M phosphate buffer containing 2 mM EDTA, pH 6.0) to give 9.8 mg of SH-introduced HRP. Next, 8 mg of the SH-introduced HRP was mixed with 3 mg of the maleimido-introduced antibody fraction. The mixture was concentrated to about 0.5 ml with Collodion Bag (manufactured by Sartorius K. K.) and the concentrate was allowed to stand at 4°C for 16 hours. The reaction solution was applied on a Sephacryl S-300HR column (manufactured by Pharmacia) using 0.1 M phosphate buffer (pH 6.5) to purify the ZAL2-103a-HRP complex fraction.

### EXAMPLE 4

### Sandwich assay-EIA

After 100 µl each of 0.1 M carbonate buffer (pH 9.6 solution) containing 15 µg/ml of the purified monoclonal antibody ZAL2-106a obtained in EXAMPLE 1 was dispensed in a 96-well microplate, the plate was allowed to stand at 4°C for 24 hours. The redundant binding sites in the wells were inactivated by adding 400 µl of Block Ace diluted with PBS to 4-fold.

To the plate prepared as described above, 100 µl of standard human ZAQL-2 or human ZAQL-1 solution diluted with 0.02 M phosphate buffer (pH 7) containing Buffer C was added, followed by reacting at 4°C for 24 hours. After washing with PBS, 100 µl of ZAL2-103a-HRP (diluted to 10,000-fold with Buffer C) prepared in EXAMPLE 3 above was added and the mixture was reacted at 4°C for 24 hours. After washing with PBS, the enzyme activity on the solid phase was assayed by the procedures described in EXAMPLE 1-(3) using TMB (3,3',5,5'-tetramethylbenzidine) Microwell Peroxidase Substrate System (Funakoshi) (enzyme reaction for 20 minutes).

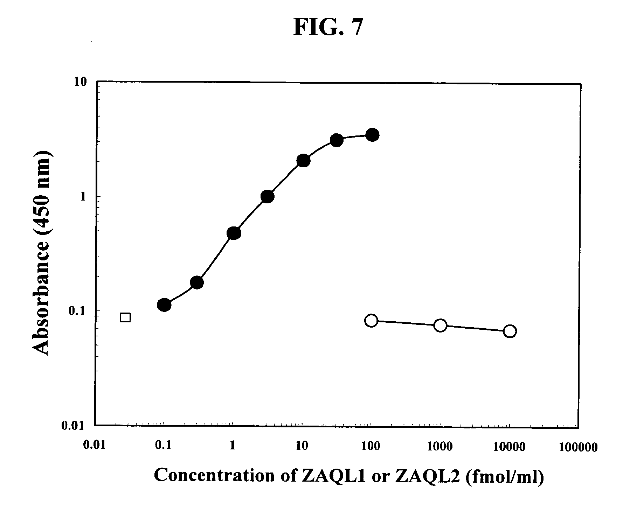

The results are shown in FIG 7.

According to the sandwich assay-EIA, human ZAQL-2 can be detected in 0.3 fmol/well and any reaction with human ZAQL-1 did not occur up to 10000 fmol/mL. Thus, it was found that the sandwich assay-EIA using ZAL2-106a as the solid-phase antibody and ZAL2-103a-HRP as the labeled antibody can detect human ZAQL-2 with an extremely high sensitivity and extremely selectively.

### EXAMPLE 5

### Neutralizing effect on biological activities of human ZAQL-2 by anti-ZAL2-monoclonal antibody

The neutralizing activities on human ZAQL-2 by ZAL2-85a, ZAL2-103a and ZAL2-106a were assayed on FLIPR (Molecular Devices) using as an indicator the intracellular Ca²⁺ ion level increasing activity in the 15E-expressed CHO cells described in EXAMPLE 3-5 of WO 02/06483.

The 15E-expressed CHO cells were suspended in dialyzed fetal bovine serum (hereinafter dFBS; JRH BIOSCIENCES)-containing Dulbecco's modified Eagle's medium (DMEM) (Nissui Pharmaceutical Co., Ltd.) in 1.2 x 10⁵ cells/ml (10% dFBS-DMEM). A 200 µl aliquot of the suspension was plated on each well of a 96-well plate (Black plate clear bottom, Coster, Inc.) for FLIPR, using a dispenser (4 x 10⁴ cells/200 µl/well), and cultured at 37°C overnight in a 5% CO₂ incubator, which was provided for use (hereinafter referred to as the cell plate). Then, 20 ml of FLIPR assay buffer [9.8 g of Nissui Hanks 2 (Nissui Pharmaceutical Co., Ltd.), 0.35 g of sodium hydrogencarbonate and 4.77 g of HEPES; adjusted to pH 7.4 with 6 M sodium hydroxide solution, made 1 liter and sterilized by filtration] was mixed with 200 µl of 250 mM Probenecid (Sigma) and 210 µl of fetal bovine serum (FBS). Also, 2 vials (50 µg) of Fluo 3-AM (Dojin Chemical Laboratory, Ltd.) was dissolved in 40 µl of dimethylsulfoxide and 40 µl of 20% Pluronic acid (Molecular Probe, Inc.). The solution was added to the H/HBSS-Probenecid-FBS solution containing 20 ml of H/HBSS (9.8 g of HEPES-buffered Hank's Balanced Salt Solution (Nissui Hanks 2 (Nissui Pharmaceutical Co., Ltd.), 0.35 g of sodium hydrogencarbonate and 4.77 g of HEPES; adjusted to pH 7.4 with a sodium hydroxide solution and then sterilized by filtration)), 200 µl of 250 mM Probenecid and 200 µl of fetal bovine serum (FBS), followed by mixing them. Using an 8 channel pipette, 100 µl each of the resulting mixture was dispensed on each well of the cell plate, from which the medium was removed. Incubation was carried out in a 5% CO₂ incubator at 37°C for an hour (dye loading). ZAL2-85a, ZAL2-103a or ZAL2-106a was diluted with 120 µl of Hanks'/HBSS supplemented with 2.5 mM Probenecid and 0.2% BSA. After incubating with human ZAQL-2 (3.3 x 10⁻¹¹ M) at 37°C for an hour, 5 µl of each fraction was transferred to a 96-well plate (V-Bottom Plate, Coster, Inc.) for FLIPR (hereinafter referred to as the sample plate). After completion of dye loading on the cell plate, the cell plate was washed 4 times with a wash buffer of Hanks'/HBSS supplemented with 2.5 mM Probenecid, using a plate washer (Molecular Devices) to retain 100 µl of the wash buffer after washing. The cell plate and the sample plate were set on FLIPR to perform assay (50 µl of a sample was transferred from the sample plate to the cell plate by FLIPR).

The results are shown in FIG 8.

The results show that ZAL2-103a inhibited the activity of human ZAQL-2 (3.3 x 10⁻¹¹ M) by about 97% in 3.3 x10⁻¹¹ M. Also, ZAL2-106a and ZAL2-85a exhibited the neutralizing activities of 95% or higher in a 10-fold concentration (3.3 x 10⁻¹⁰ M) and in a 100-fold concentration (3.3 x 10⁻⁹ M), respectively.

The foregoing results reveal that especially ZAL2-103a neutralized the intracellular Ca²⁺ increasing activity in human ZAQL-2 in an equimolar concentration to human ZAQL-2, indicating that ZAL2-103a is usable as a neutralizing antibody.

### EXAMPLE 6

### Quantification of human ZAQL-2 in plasma

Human plasma was diluted to 2-fold with an equal volume of Buffer C. Human ZAQL-2 was quantified by the sandwich assay-EIA in EXAMPLE 4 described above.

The results are shown in TABLE 2.

**TABLE 2**

| Immunoreactivity of Human ZAQL-2 in Plasma | | |
|---|---|---|
| No. | Male (fmol/ml) | Female (fmol/ml) |
| 1 | 2.41 | 5.68 |
| 2 | 3.42 | 53.54 |
| 3 | 4.56 | 0.38 |
| 4 | 1.69 | 0.35 |
| 5 | 0.94 | 6.84 |
| 6 | 1.23 | 4.45 |
| 7 | 1.46 | 6.39 |
| 8 | 1.45 | 1.54 |
| 9 | 0.83 | |
| 10 | 1.53 | |

Concentration of human ZAQL-2 in human plasma (1 ml)
Male: 1.95 ± 0.38 fmol/ml (mean ± SEM, n=10)
Female: 9.90 ± 6.30 fmol/ml (mean ± SEM, n=8)

The results reveal that this assay system can accurately quantify the changes of human ZAQL-2 in plasma.

### EXAMPLE 7

### Detection of human ZAQL-2 in human plasma by reversed phase high performance liquid chromatography

To quantify the immunoreactivity of human ZAQL-2 contained in human plasma, which was described in EXAMPLE 6, 5 ml of acetonitrile was added to 2.5 ml of human plasma followed by mixing them. The mixture was centrifuged (15,000 rpm, 5 minutes) to remove proteins. After the supernatant was lyophilized, this fraction was concentrated and the concentrate was fractionated on reversed phase HPLC using ODS-80™.
Column conditions:
Column: ODS-80™ (4.6 x 250 mm)
Eluants: Eluant A (5% acetonitrile containing 0.05% trifluoroacetic acid)

Eluant B (60% acetonitrile containing 0.05% trifluoroacetic acid)
Elution method: The acetonitrile concentration was increased from 5% to 30% for the initial 5 minutes and then linearly increased to 30-40% over 30 minutes.
Flow rate: 1.0 ml/min.
Fractionation: 0.5 ml/tube

After the eluted fraction was lyophilized, the lyophilized product was dissolved in 250 µl of Buffer C and the solution was provided for the sandwich assay-EIA described in EXAMPLE 4.

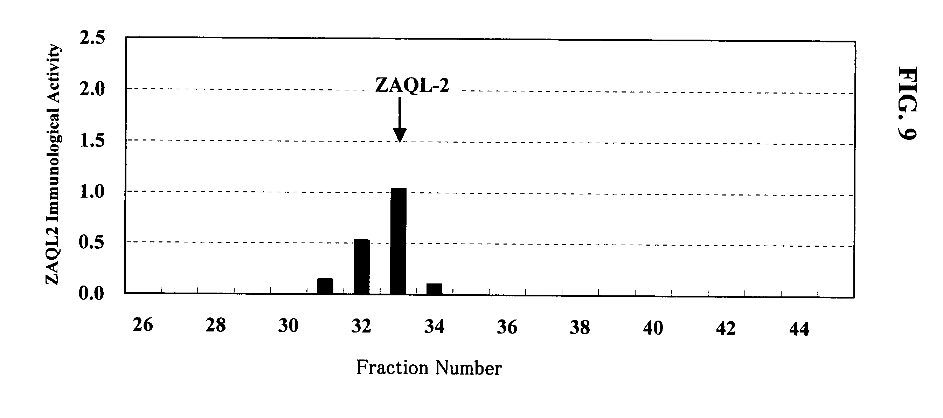

The results are shown in FIG. 9. The immunoreactivity of human ZAQL-2 in plasma was detected almost at the eluted position of human ZAQL-2 in plasma (recovery rate of 66%). It was thus confirmed that the sandwich assay-EIA detected human ZAQL-2.

These results reveal that this assay system can be an important tool to study the changes of human ZAQL-1 in plasma.

### INDUSTRIAL APPLICABILITY

The antibody of the present invention possesses extremely high binding ability to the polypeptide comprising the amino acid sequence represented by SEQ ID NO: 1, or salts thereof and can also neutralize the intracellular [Ca²⁺] increasing activity of the polypeptide comprising the amino acid sequence represented by SEQ ID NO: 1, or salts thereof. By suppressing (or promoting) the actions of the polypeptide comprising the amino acid sequence represented by SEQ ID NO: 1, or salts thereof, the antibody of the present invention is useful as an agent for the prevention and/or treatment of, for example, digestive diseases (e.g., enteritis, diarrhea, constipation, malabsorption syndrome, etc.), sleeping disorders (e.g., primary insomnia, circadian rhythm disorders (e.g., change in physical conditions caused by three-shift work, time zone change syndrome (jet lag), etc.), narcolepsy, etc.), seasonal depression, reproductive dysfunction (e.g., endometriosis, etc.), endocrine diseases, central nervous system disorders (e.g., senile dementia, Alzheimer's disease, parkinsonian syndrome, etc.), cerebral circulatory disorders (e.g., apoplexy, etc.), various disorders caused by aging, mental disorders (e.g., anxiety, depression, insomnia, schizophrenia, phobia, etc.), memory impairment, motor dysfunction (e.g., parkinsonian syndrome, etc.),epilepsy, alcoholism, hypertension, arteriosclerosis, arrhythmia, premenstrual syndrome, glaucoma, cancer(e.g., breast cancer, etc.), AIDS, diabetes mellitus, eating disorders (e.g., anorexia, bulimia, etc.), obesity (e.g., malignant mastocytosis, exogenous obesity, hyperinsulinar obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity, etc.), osteoporosis, etc. Preferably, the antibody is useful as an agent for the prevention and/or treatment of sleeping disorders, reproductive dysfunction, endocrine diseases, central nervous system disorders, mental disorders, motor dysfunction, eating disorders, etc., and more preferably, as an agent for the prevention and/or treatment of endocrine diseases, central nervous system disorders, motor dysfunction, etc. Also, when cancers where the polypeptide comprising the amino acid sequence represented by SEQ ID NO: 1, or salts thereof are expressed are identified, an anti-cancer treatment can be performed by missile therapy using the antibody of the present invention. In addition, the polypeptide comprising the amino acid sequence represented by SEQ ID NO: 1, or salts thereof can be quantified with a high sensitivity by the sandwich immunoassay using the two antibodies of the present invention and is thus useful for clarification of the physiological functions and pathological conditions of the polypeptide comprising the amino acid sequence represented by SEQ ID NO: 1, or salts thereof. Moreover, the diseases described above can also be diagnosed by determining the level of the polypeptide comprising the amino acid sequence represented by SEQ ID NO: 1, or salts thereof in blood. Furthermore, the antibody of the present invention is available for immunohistochemical staining of the polypeptide described above.

## Claims

1. A monoclonal antibody reacting specifically with a polypeptide comprising the amino acid sequence represented by SEQ ID NO: 1, or a salt thereof.

2. The monoclonal antibody according to claim 1, which reacts specifically with a peptide comprising at least one sequence selected from the amino acid sequences of the 8th to 9th, 11th, 15th, 17th, 21st, 23rd, 25th to 28th, 30th, 34th, 36th to 37th, 39th to 40th, 44th to 46th, 48th, 52nd-53rd, 55th, 64th, 66th, 68th, 70th to 73rd, 75th to 76th and 78th to 81st in the amino acid sequence represented by SEQ ID NO: 1.

3. The monoclonal antibody according to claim 1, which does not recognize a polypeptide comprising the amino acid sequence represented by SEQ ID NO: 2 or SEQ ID NO: 3.

4. The monoclonal antibody according to claim 1, which is labeled.

5. The monoclonal antibody according to claim 1, which is shown by ZAL2-103a producible from the hybridoma shown by ZAL2-103 (FERM BP-8431).

6. The monoclonal antibody according to claim 1, which is shown by ZAL2-106a producible from the hybridoma shown by ZAL2-106 (FERM BP-8432).

7. The antibody according to claim 1, which has a neutralizing activity to a peptide having the amino acid sequence represented by SEQ ID NO: 1.

8. A pharmaceutical composition comprising the monoclonal antibody according to claim 1.

9. A diagnostic agent comprising the monoclonal antibody according to claim 1.

10. A method of quantifying a polypeptide comprising the amino acid sequence represented by SEQ ID NO: 1, which comprises using the monoclonal antibody according to claim 1.

11. A method of diagnosing a disease associated with a polypeptide comprising the amino acid sequence represented by SEQ ID NO: 1, or a salt thereof, which comprises using the monoclonal antibody according to claim 1.

12. A method of quantifying a polypeptide comprising the amino acid sequence represented by SEQ ID NO: 1, or a salt thereof in a test fluid, which comprises competitively reacting the monoclonal antibody according to claim 1 with a test fluid and a labeled form of polypeptide comprising the amino acid sequence represented by SEQ ID NO: 1, or a salt thereof, and measuring a ratio of the labeled polypeptide or a salt thereof bound to said antibody.

13. A method of quantifying a polypeptide comprising the amino acid sequence represented by SEQ ID NO: 1, or a salt thereof in a test fluid, which comprises reacting the monoclonal antibody according to claim 5 immobilized on a carrier, a labeled form of the monoclonal antibody according to claim 6 and a test fluid, and then assaying the activity of a labeling agent.

14. A method of quantifying a polypeptide comprising the amino acid sequence represented by SEQ ID NO: 1, or a salt thereof in a test fluid, which comprises reacting the monoclonal antibody according to claim 6 immobilized on a carrier, a labeled form of the monoclonal antibody according to claim 5 and a test fluid, and then assaying the activity of a labeling agent.

15. A hybridoma producing the monoclonal antibody according to claim 1.

16. The hybridoma according to claim 15, which is shown by ZAL2-103 (FERM BP-8431) or ZAL2-106 (FERM BP-8432).

17. A method of producing the monoclonal antibody according to claim 1, which comprises culturing the hybridoma according to claim 15 in vivo or in vitro and collecting the monoclonal antibody according to claim 1 from the body fluid or culture.

18. The pharmaceutical according to claim 8, which is an agent for the prevention and/or treatment of central nervous system disorders, motor dysfunction or endocrine diseases.

19. The diagnostic agent according to claim 9, which is a diagnostic agent for central nervous system disorders, motor dysfunction or endocrine diseases.

20. An antibody reacting specifically with the binding site of the antibody according to claim 1 against a polypeptide comprising the amino acid sequence represented by SEQ ID NO: 1.

21. An antibody reacting specifically with the binding site of the antibody according to claim 5 against a polypeptide comprising the amino acid sequence represented by SEQ ID NO: 1.

22. An antibody reacting specifically with the binding site of the antibody according to claim 6 against a polypeptide comprising the amino acid sequence represented by SEQ ID NO: 1.

23. A method of preventing and/or treating central nervous system disorders, motor dysfunction or endocrine diseases, which comprises administering to a mammal an effective dose of the antibody according to claim 1.

24. Use of the antibody according to claim 1 to manufacture an agent for the prevention and/or treatment of central nervous system disorders, motor dysfunction or endocrine diseases.


SEQUENCE LISTING

<110> Takada Pharmaceutical Company Limited

<120> Antibody and its use

<130> G06-0003

<150> JP2003-361639
<151> 2003-10-22

<160> 3

<210> 1
<211> 81
<212> PRT
<213> Human

<400> 1
Ala Val Ile Thr Gly Ala Cys Asp Lys Asp Ser Gln Cys Gly Gly Gly
                5                   10                  15
Met Cys Cys Ala Val Ser Ile Trp Val Lys Ser Ile Arg Ile Cys Thr
            20                  25                  30
Pro Met Gly Lys Leu Gly Asp Ser Cys His Pro Leu Thr Arg Lys Val
        35                  40                  45
Pro Phe Phe Gly Arg Arg Met His His Thr Cys Pro Cys Leu Pro Gly
    50                  55                  60
Leu Ala Cys Leu Arg Thr Ser Phe Asn Arg Phe Ile Cys Leu Ala Gln
65                  70                  75                  80
Lys

<210> 2
<211> 86
<212> PRT
<213> Human

<400> 2
Ala Val Ile Thr Gly Ala Cys Glu Arg Asp Val Gln Cys Gly Ala Gly
                5                   10                  15
Thr Cys Cys Ala Ile Ser Leu Trp Leu Arg Gly Leu Arg Met Cys Thr
            20                  25                  30
Pro Leu Gly Arg Glu Gly Glu Glu Cys His Pro Gly Ser His Lys Val
        35                  40                  45
Pro Phe Phe Arg Lys Arg Lys His His Thr Cys Pro Cys Leu Pro Asn
    50                  55                  60
Leu Leu Cys Ser Arg Phe Pro Asp Gly Arg Tyr Arg Cys Ser Met Asp
65                  70                  75                  80
Leu Lys Asn Ile Asn Phe
                85

<210> 3
<211> 86
<212> PRT
<213> Human

<400> 3
Ala Val Ile Thr Gly Ala Cys Glu Arg Asp Val Gln Cys Gly Ala Gly
                5                   10                  15
Thr Cys Cys Ala Ile Ser Leu Trp Leu Arg Gly Leu Arg Met Cys Thr
            20                  25                  30
Pro Leu Gly Arg Glu Gly Glu Glu Cys His Pro Gly Ser His Lys Ile
        35                  40                  45
Pro Phe Phe Arg Lys Arg Lys His His Thr Cys Pro Cys Leu Pro Asn
    50                  55                  60
Leu Leu Cys Ser Arg Phe Pro Asp Gly Arg Tyr Arg Cys Ser Met Asp
65                  70                  75                  80
Leu Lys Asn Ile Asn Phe
                85